# FASCICULE DE BREVET EUROPEEN

(11) **EP 3 036 546 B1**
(45) Date de publication et mention de la délivrance du brevet: **22.11.2017**
(21) Numéro de dépôt: 14767128.3
(22) Date de dépôt: 25.07.2014
(51) Int. Cl.: G01N 15/14, B01L 3/00, G01N 33/543, G01N 33/569

(54) **SYSTÈME MICROFLUIDIQUE ET PROCÉDÉ POUR ISOLER ET QUANTIFIER AU MOINS UNE SOUS-POPULATION DE CELLULES À PARTIR D'UNE POPULATION DE CELLULES**
MIKROFLUIDISCHES SYSTEM UND VERFAHREN ZUR ISOLIERUNG UND QUANTIFIZIERUNG VON MINDESTENS EINER SUBPOPULATION VON ZELLEN AUS EINER ZELLPOPULATION
MICROFLUIDIC SYSTEM AND METHOD FOR ISOLATING AND QUANTIFYING AT LEAST ONE SUB-POPULATION OF CELLS FROM A POPULATION OF CELLS

(30) Priorité: 26.07.2013 FR 1357410
(43) Date de publication de la demande: 29.06.2016
(73) Titulaire: Centre National de la Recherche Scientifique, 75016 Paris (FR)
(72) Inventeur: GUE, Anne-Marie, F-31320 Rebigue (FR); REYBIER, Karine, 31320 Aureville (FR); SUDOR, Jan, F-31500 Toulouse (FR); CARGOU, Sébastien, 75003 Paris (FR); MONTROSE, Armelle, 93110 Rosny Sous Bois (FR)
(74) Mandataire: Gevers & Orès
(86) Numéro de dépôt international: PCT/IB2014/063406
(87) Numéro de publication internationale: WO 2015/011674

(56) Documents cités:
- WO-A2-2007/092713
- US-A1- 2011 129 931
- Armelle Montrose: "Développement d'un immunocapteur impédimétrique pour la détection et la quantification d'une sous- population cellulaire : Application au diagnostic précoce des infections", , 22 mars 2013 (2013-03-22), XP055110165, Extrait de l'Internet: URL:http://thesesups.ups-tlse.fr/1963/1/20 13TOU30033.pdf [extrait le 2014-03-26]
- Hiromichi Inokuchi ET AL: "Development of micro immuno-magnetic cell sorting system with lamination mixer and magnetic separator", Proc. 25th Sensor Symp. (Okinawa, Japan, 2008), 1 janvier 2008 (2008-01-01), XP055110832, Extrait de l'Internet: URL:http://thtlab.jp/Doc/inokuchi-sensor08 .pdf [extrait le 2014-03-31]
- SHEW B Y ET AL: "Enhancement of specific cell-capture efficiency using a reversible dielectrophoresis field", SENSORS AND ACTUATORS A, ELSEVIER SEQUOIA S.A., LAUSANNE, CH, vol. 163, no. 1, 1 septembre 2010 (2010-09-01), pages 128-137, XP027383669, ISSN: 0924-4247 [extrait le 2010-08-11]
- CHUNG Y K ET AL: "An electrical biosensor for the detection of circulating tumor cells", BIOSENSORS AND BIOELECTRONICS, ELSEVIER BV, NL, vol. 26, no. 5, 15 janvier 2011 (2011-01-15), pages 2520-2526, XP027580192, ISSN: 0956-5663 [extrait le 2010-11-04]
- ARMELLE MONTROSE ET AL: "Impedimetric immunosensor for the detection of circulating pro-inflammatory monocytes as infection markers", BIOSENSORS AND BIOELECTRONICS, vol. 49, 25 mai 2013 (2013-05-25), pages 305-311, XP055110167, ISSN: 0956-5663, DOI: 10.1016/j.bios.2013.05.025
- YONGMO YANG ET AL: "Separating and Detecting Escherichia Coli in a Microfluidic Channel for Urinary Tract Infection Applications", JOURNAL OF MICROELECTROMECHANICAL SYSTEMS, IEEE SERVICE CENTER, US, vol. 20, no. 4, 1 août 2011 (2011-08-01), pages 819-827, XP011338232, ISSN: 1057-7157, DOI: 10.1109/JMEMS.2011.2159095

## Description

La présente invention concerne un système microfluidique apte à recevoir des populations de cellules et apte à isoler et à quantifier simultanément pour chacune des populations au moins une sous-population de cellules, et un procédé d'isolement et de quantification simultanés d'au moins une sous-population de cellules à partir d'une population de cellules utilisant ce système. L'invention s'applique en particulier au tri de cellules d'intérêt constituées de monocytes du sang dits « circulants » à partir de populations de cellules formées d'échantillons sanguins, suivi d'un isolement et d'une quantification d'au moins l'une de ces sous-populations formées de monocytes infectés (i.e. marqueurs d'inflammations). Cependant, l'invention s'applique d'une manière générale au tri immunologique et au comptage de toute cellule circulante (e.g. des cellules cancéreuses ou des cellules, marqueurs de maladies tropicales et infectieuses) ou de toute cellule obtenue par prélèvement d'un tissu et mise en solution par un seul et même système intégrant les techniques de la micro- ou nanotechnologie, par exemple pour le diagnostic médical rapide et rapproché, le secteur vétérinaire ou même de la défense, à titre non limitatif.

Il est connu d'intégrer à un système microfluidique des moyens de piégeage magnétique de composés biologiques préalablement liés à des microbilles magnétiques fonctionnalisées, ces moyens de piégeage étant tels que des électroaimants sous forme de micro-bobines obtenues par électrodéposition ou bien sous forme de tout autre conducteur électrique comme par exemple des serpentins ou des fils organisés en matrices. On peut par exemple citer pour un tel système l'article de R. Fulcrand, D. Jugieu, C. Escriba, A. Bancaud, D. Bourrier, A. Boukabache et A. M. Gué, Development of a flexible microfluidic system integrating magnetic micro-actuators for trapping biological species, Journal of Micromechanics and Microengineering 19 (2009) 105019.

Il est également connu d'intégrer à un système microfluidique un compteur différentiel pour la quantification sélective de cellules, telles que des lymphocytes comptés à partir d'une population de leucocytes présente dans des échantillons de sang humain, par mesure d'impédance et chromatographie d'immunoaffinité. On peut par exemple citer pour un tel système l'article de Mishra N. N., Retterer S., Zieziulewicz T. J., lsaacson M., Szarowski D., Mousseau D. E., Lawrence D. A., Turner J. N., On~chip micro-biosensor for the detection of human CD4(+) cells based on AC impedance and optical analysis, Biosens Bioelectron. 2005 Nov. 15; 21 (5): 696-704.

On a par ailleurs développé par le passé des systèmes pour réaliser à la fois le tri et le comptage cellulaires, qui utilisent les techniques de FACS (« fluorescence-activated cell sorting » en anglais, pour tri de cellules activé par fluorescence) et de cytométrie de flux.

Un inconvénient majeur de ces derniers systèmes de tri et de comptage réside dans les contraintes liées à leur utilisation, qui requièrent notamment des appareillages lourds et volumineux, un temps nécessaire à l'analyse relativement long et des personnels qualifiés. Il en résulte que ces systèmes présentent un coût d'utilisation élevé, et sont de ce fait inadaptés à un suivi régulier et rapproché de patients ou à une utilisation « sur le terrain », i.e. au chevet du patient, dans un cabinet médical, une clinique vétérinaire ou en zone tropicale, par exemple.

Le document de Thèse d'Armelle Montrose paru sur internet : « Développement d'un immunocapteur impédimétrique pour la détection et la quantification d'une sous-population cellulaire: application au diagnostic précoce des infections », 22 mars 2013, présente un système microfluidique et un procédé d'isolement et de quantification simultanés d'une sous-population de cellules utilisant une première unité de tri pour isoler par attraction magnétique des cellules d'intérêt dans un microcanal de premier tri, et une seconde unité de tri et de comptage simultanés qui comporte un microcanal communicant de second tri à électrode fonctionnalisée pour piéger une sous-population de cellules et qui comporte des moyens de comptage par spectroscopie d'impédance électrochimique de la sous-population piégée sur l'électrode fonctionnalisée.

La demande de brevet US2011/0129931 A1 présente un système microfluidique utilisant un moyen de piégeage magnétique de cellules marquées avec des billes magnétiques sur des électrodes fonctionnalisées pour une détection par impédance.

Un but de la présente invention est de proposer un système microfluidique apte à recevoir des populations de cellules et apte à isoler et à quantifier simultanément pour chacune desdites populations au moins une sous-population de cellules, ledit système comportant un substrat dans lequel est gravé un réseau de microcanaux comprenant une première unité de tri apte à isoler par attraction magnétique des cellules d'intérêt de ladite population de cellules dans au moins un microcanal de premier tri, ce système remédiant à l'ensemble des inconvénients précités.

Ce système microfluidique est tel que ledit réseau comprend une seconde unité de tri et de comptage simultanés comportant au moins un microcanal de second tri qui communique directement ou indirectement avec ledit au moins un microcanal de premier tri et qui est défini par une paroi fermée présentant une face interne destinée à être en contact avec lesdites cellules d'intérêt, ladite face interne étant pourvue d'au moins une électrode fonctionnalisée apte à piéger spécifiquement une dite sous-population de cellules parmi lesdites cellules d'intérêt, ladite seconde unité de tri et de comptage comportant des moyens de comptage par spectroscopie d'impédance électrochimique de ladite ou chaque sous-population piègée sur ladite au moins une électrode fonctionnalisée.

A cet effet, un système microfluidique selon l'invention est tel que ledit au moins un microcanal de second tri est pourvu d'au moins une paire de ces électrodes fonctionnalisées agencées en regard l'une de l'autre en deux côtés opposés de ladite face interne, et d'au moins une paire associée de secondes micro-bobines de piégeage alimentées électriquement qui sont aptes à amener les cellules d'intérêt en contact étroit avec ces électrodes fonctionnalisées, qui sont agencées dans ladite paroi de manière adjacente et en regard desdites électrodes respectives (i.e. agencées en face et juste en dessous des faces des électrodes adjacentes tournées vers cette paroi) et qui sont aptes à commander des cycles d'attraction et de libération successifs et alternés entre les micro-bobines de ladite au moins une paire de secondes micro-bobines.

On notera que ce système microfluidique selon l'invention, de type laboratoire sur puce, permet ainsi sur une même puce d'isoler et de compter simultanément de manière aisée ces sous-populations cellulaires directement par piégeage sur ces électrodes (ce comptage étant réalisé par des mesures d'impédance à l'interface cellules piégées / électrode(s) fonctionnalisée(s) pour une large gamme de fréquences), en comparaison des systèmes précités de l'art antérieur utilisant les techniques lourdes et coûteuses de « FAGS » et de cytométrie de flux pour le tri et le comptage. En particulier, la miniaturisation du système selon l'invention et l'intégration totale des moyens de comptage à ce système fait que ce dernier est très bien adapté aux secteurs pour lesquels le recours à la technique « FACS » s'avère beaucoup trop coûteux ou inadapté, comme par exemple le diagnostic médical rapide et rapproché, le secteur vétérinaire ou la défense.

Selon une autre caractéristique de l'invention, ladite première unité de tri peut comprendre :
- un microcanal d'amenée d'une dite population de cellules qui débouche dans ledit au moins un microcanal de premier tri,
- un microcanal d'introduction de moyens magnétiques de marquage desdites cellules d'intérêt qui débouche dans ledit au moins un microcanal de premier tri et dans lequel sont introduits lesdits moyens magnétiques de marquage capables de se lier spécifiquement avec lesdites cellules d'intérêt pour former des complexes magnétiques marqués, et
- des moyens de piégeage magnétique qui sont agencés sous ledit au moins un microcanal de premier tri et qui sont capables d'attirer et de retenir lesdits complexes magnétiques marqués.

Avantageusement, lesdites moyens magnétiques de marquage peuvent comprendre des microbilles ou des nanobilles magnétiques fonctionnalisées par une première substance apte à reconnaître spécifiquement un premier marqueur desdites cellules d'intérêt, et lesdits moyens de piégeage magnétique peuvent comprendre au moins une première micro-bobine alimentée électriquement.

On notera qu'en variante, ces moyens de piégeage magnétique selon l'invention peuvent comprendre tout autre conducteur électrique comme par exemple des serpentins ou des fils organisés en matrices, à titre non limitatif.

Egalement avantageusement, lesdites électrodes fonctionnalisées peuvent être fonctionnalisées par une seconde substance capable de se lier spécifiquement avec un second marqueur de ladite sous-population à isoler parmi lesdites cellules d'intérêt.

On notera que le système microfluidique de l'invention utilise ainsi une différenciation basée sur l'expression d'au moins deux marqueurs (e.g. des antigènes ou récepteurs caractéristiques des cellules d'intérêt que l'on souhaite isoler par exemple pour la détection d'un état infectieux), en combinant dans ce même système le marquage des cellules d'intérêt par lesdits moyens magnétiques de marquage, le tri magnétique de ces cellules d'intérêt par lesdits moyens de piégeage, et un tri immunologique avec comptage simultané de la ou de chaque sous-population triée parmi les cellules d'intérêt en ce second niveau de tri.

Outre le fait que le système microfluidique de l'invention peut être avantageusement utilisé dans un très grand nombre d'applications, comme indiqué ci-dessus en préambule et qu'il autorise une réduction du volume d'échantillons nécessaire, à l'analyse en comparaison d'un système non microfluidique, on notera que ce système se caractérise notamment par le couplage tri/ comptage qui lui confère de multiples avantages, parmi lesquels:
- une grande généricité, étant précisé qu'il peut y avoir dans ce système autant de critères de tri que de marqueurs immunologiques,
- une très grande flexibilité, grâce à la miniaturisation du système qui permet, comme cela sera exposé ci-après, la parallélisation du tri et du comptage sur la base de critères immunologiques différents, et/ou l'enchaînement d'étapes de tri successives pour affiner un diagnostic,
- un très faible encombrement, du fait que le comptage de type électrique mis en oeuvre par le système selon l'invention n'impose pas d'associer une instrumentation lourde à la seconde unité de tri ce qui peut conférer au système un format sensiblement aussi réduit que celui d'un téléphone mobile, rendant ce système portable, autonome en énergie et éventuellement communicant,
- une aptitude à détecter de très petits nombres de cellules dans la ou dans chaque sous-population de cellule finalement isolée (e.g. de faibles nombres de cellules infectées dans un très grand nombre de cellules normales), et
- des temps d'analyse réduits, puisque le comptage de la ou de chaque sous-population de cellules est collectif.

Selon un mode avantageux de réalisation de l'invention, ladite face interne de paroi dudit au moins un microcanal de second tri est pourvue de manière espacée sur sa longueur d'une succession de plusieurs électrodes incluant ladite au moins une paire d'électrodes fonctionnalisées puis une électrode non fonctionnalisée ou paire d'électrodes non fonctionnalisées agencées en regard l'une de l'autre en deux côtés opposés de ladite face interne, pour réaliser des isolements et quantifications en série de plusieurs dites sous-populations de cellules.

Conformément à ce mode de réalisation de l'invention, ladite seconde unité de tri et de comptage peut comprendre une pluralité de dites paires d'électrodes fonctionnalisées qui sont agencées en série (i.e. à la suite l'une de l'autre) sur un même dit microcanal de second tri ou bien sur plusieurs dits microcanaux de second tri, qui sont fonctionnalisées de manière identique ou non et qui sont associées à plusieurs dites secondes micro-bobines de piégeage individuelles.

Avantageusement et optionneliement suivant ce mode de réalisation de l'invention, ladite première unité de tri peut comprendre une pluralité de dits microcanaux de premier tri, et ladite seconde unité de tri et de comptage peut comprendre une pluralité de dits microcanaux de second tri qui sont chacun pourvus de ladite au moins une paire d'électrodes fonctionnalisées, de telle sorte que ledit système réalise en parallèle une pluralité d'isolements et de quantifications différents de dites sous-populations de cellules à partir de différentes cellules d'intérêt

On peut ainsi marquer en parallèle (i.e. dans des microcanaux de premier tri différents) les cellules d'intérêt par les microbilles ou nanobilles magnétiques fonctionnalisées différemment et ainsi compter en parallèle des marqueurs différents (i.e. dans des microcanaux de second tri différents).

Inversement, on notera qu'il est possible de marquer et de « démarquer » successivement les cellules d'intérêt pour comptabiliser celles ayant exprimé plusieurs marqueurs.

Encore plus avantageusement en relation avec cette réalisation en parallèle de différents isolements et quantifications, ladite seconde unité de tri et de comptage peut comprendre une pluralité de dites paires d'électrodes fonctionnalisées qui sont agencées en parallèle (i.e. indépendamment l'une de l'autre) sur un même dit microcanal de second tri ou bien sur plusieurs dits microcanaux de second tri, qui sont fonctionnalisées de manière identique ou non et qui sont associées à autant de dites paires de secondes micro-bobines de piégeage individuelles.

On notera que les systèmes microfluidiques selon l'invention peuvent être équipés de manière flexible en fonction des analyses à réaliser d'un nombre variable desdites première(s) et secondes micro-bobines et desdites électrodes fonctionnalisées et non fonctionnalisée(s), lesquelles micro-bobines et électrodes peuvent présenter des géométries et localisations également variables au sein du réseau de microcanaux qui peut être ramifié en conséquence.

Selon une variante de l'invention, ladite seconde unité de tri et de comptage peut comprendre une matrice de dites paires d'électrodes fonctionnalisées qui sont adressées individuellement, et qui sont associées à autant de dites secondes micro-bobines de piégeage individuelles.

On notera que cette matrice d'électrodes permet d'optimiser la sensibilité du comptage en l'adaptant à l'analyse souhaitée, en comparaison d'une unique électrode ou paire d'électrodes de grande(s) surface(s).

Un procédé selon l'invention d'isolement et de quantification simultanés d'au moins une sous-population de cellules à partir d'une population de cellules, est caractérisé en ce qu'il comprend un écoulement d'un échantillon comprenant ladite population de cellules dans un réseau de microcanaux d'un système microfluidique tel que défini ci-dessus, avec les étapes successives suivantes :
a) un piégeage par attraction magnétique desdites cellules d'intérêt dans ledit au moins un microcanal de premier tri,
b) une élimination des constituants indésirables dudit échantillon pour ne conserver que lesdites cellules d'intérêt ainsi piégées, puis
c) un piégeage de ladite au moins une sous-population de cellules parmi lesdites cellules d'intérêt dans ledit au moins un microcanal de second tri par ladite au moins une paire d'électrodes fonctionnalisées, et un comptage concomitant par spectroscopie d'impédance électrochimique de ladite au moins une sous-population piégée.

Selon une autre caractéristique de l'invention, à l'étape a) :
- on peut marquer lesdites cellules d'intérêt par des microbilles ou nanobilles magnétiques fonctionnalisées par une première substance se liant spécifiquement avec un premier marqueur desdites cellules d'intérêt, pour l'obtention de complexes magnétiques marqués, puis
- on peut piéger magnétiquement lesdits complexes par au moins une première micro-bobine.

Selon une autre caractéristique de l'invention, à l'étape c), on peut attirer lesdits complexes magnétiques marqués par au moins une paire de secondes micro-bobines de piégeage, qui est agencée en regard de ladite au moins une paire d'électrodes fonctionnalisées par une seconde substance se liant spécifiquement avec un second marqueur de ladite au moins une sous-population à isoler, et qui amène lesdits complexes en contact étroit avec ladite au moins une paire d'électrodes fonctionnalisées pour le piégeage et le comptage de cette sous-population.

Avantageusement, ledit échantillon contenant ladite population de cellules est un échantillon de sang total, lesdites cellules d'intérêt étant des monocytes que l'on isole magnétiquement à l'étape a) avec ledit premier marqueur exprimé par les cellules d'intérêt qui est un antigène par exemple CD14 et ladite première substance de marquage qui est un anticorps par exemple anti-CD14, ladite sous-population de cellules isolée et quantifiée par un tri immunologique à l'étape c) comprenant des monocytes infectés exprimant ledit second marqueur qui est un antigène par exemple CD16, ladite seconde substance étant un anticorps par exemple anti-CD16.

On notera que la détection d'un état infectieux constitue une application préférentielle du procédé selon l'invention, et qu'elle est réalisée à partir d'échantillons sanguins via l'analyse quantitative de monocytes circulants infectés comme marqueurs de l'infection. En effet, les monocytes du sang dits « circulants », qui appartiennent à la première ligne de défense contre les infections, se subdivisent de manière connue en deux groupes principaux suivant leur expression en récepteur CD14 (récepteur du lipopolysaccharide LPS) et en récepteur CD16 (récepteur Fcγ RIII de faible activité), étant rappelé que les deux principaux phénotypes de monocytes sont les CD14++ CD16- et CD14+ CD16+ et que le phénotype CD14+ CD16+ a clairement été identifié comme étant pro-inflammatoire (expression des cytokines pro-inflammatoires).

Le comptage par spectroscopie d'impédance électrochimique de l'étape c) du procédé selon l'invention est réalisé en mode non faradique, c'est-à-dire en l'absence de sonde redox, ou bien en mode faradique.

D'autres avantages, caractéristiques et détails de l'invention ressortiront du complément de description qui va suivre en référence à des dessins annexés, donnés uniquement à titre d'exemples et dans lesquels :
la figure 1 est un diagramme illustrant les principales étapes d'un procédé selon l'invention pour l'isolement et la quantification de monocytes infectés à partir d'un échantillon de sang total,
la figure 2 est une vue schématique partielle en coupe longitudinale d'un système microfluidique selon l'invention montrant une partie amont de la première unité de tri réalisant le marquage des cellules d'intérêt (e.g. des monocytes) par les moyens de marquage magnétiques,
la figure 3 est une vue schématique partielle en coupe longitudinale d'un système microfluidique selon l'invention montrant un microcanal de premier tri de la première unité de tri réalisant le tri par attraction magnétique de ces cellules d'intérêt,
la figure 4 est une vue schématique partielle en coupe longitudinale d'un système microfluidique selon l'invention montrant un microcanal de second tri réalisant l'isolement et le comptage d'une sous-populations de cellules (e.g. de monocytes infectés) sur une paire d'électrodes fonctionnalisées puis des autres cellules d'intérêt sur une électrode non fonctionnalisée,
la figure 5 est un diagramme illustrant de manière schématique la méthode de comptage par spectroscopie d'impédance électrochimique de la sous-population piégée dans ce microcanal de second tri,
la figure 6 est une vue schématique partielle en coupe transversale d'un système microfluidique selon un exemple de l'invention montrant ce microcanal de second tri pourvu d'une micro-bobine associée à une électrode fonctionnalisée pour le piégeage de la sous-population,
la figure 7 est une vue schématique partielle en coupe transversale d'un système microfluidique selon un autre exemple de l'invention montrant, en variante de la figure 6, le microcanal de second tri pourvu de deux micro-bobines associées à l'électrode fonctionnalisée de piégeage,
la figure 8 est une vue schématique montrant, dans un microcanal de second tri, un agencement en paralléle d'électrodes fonctionnalisées associées à une unique micro-bobine de piégeage collective,
la figure 9 est une vue schématique montrant en variante de la figure 8, dans un microcanal de second tri, un agencement en série d'électrodes fonctionnalisées associées a une unique micro-bobine de piégeage collective,
la figure 10 est une vue schématique montrant en variante des figures 8 et 9, dans un microcanal de second tri, une matrice d'électrodes fonctionnalisées selon l'invention qui sont adressées individuellement et qui sont respectivement associées à des micro-bobines de piégeage,
la figure 11 est une vue schématique montrant en variante de la figure 10, dans un microcanal de second tri, une matrice d'électrodes fonctionnalisées qui sont adressées individuellement et qui sont associées à une unique micro-bobine de piégeage collective,
la figure 12 est un graphique à barres illustrant l'influence de la surface (aire en cm² en abscisse) d'une électrode fonctionnalisée selon l'invention sur la sensibilité de la mesure de comptage (exprimée en ordonnée par la variation relative d'impédance ΔZ en %), et
la figure 13 est un graphique de type diagramme de Nyquist illustrant la variation d'impédance imaginaire (Zᵢₘ, en Ohms) en fonction de l'impédance réelle (Zr, en Ohms) et de la concentration en cellules,

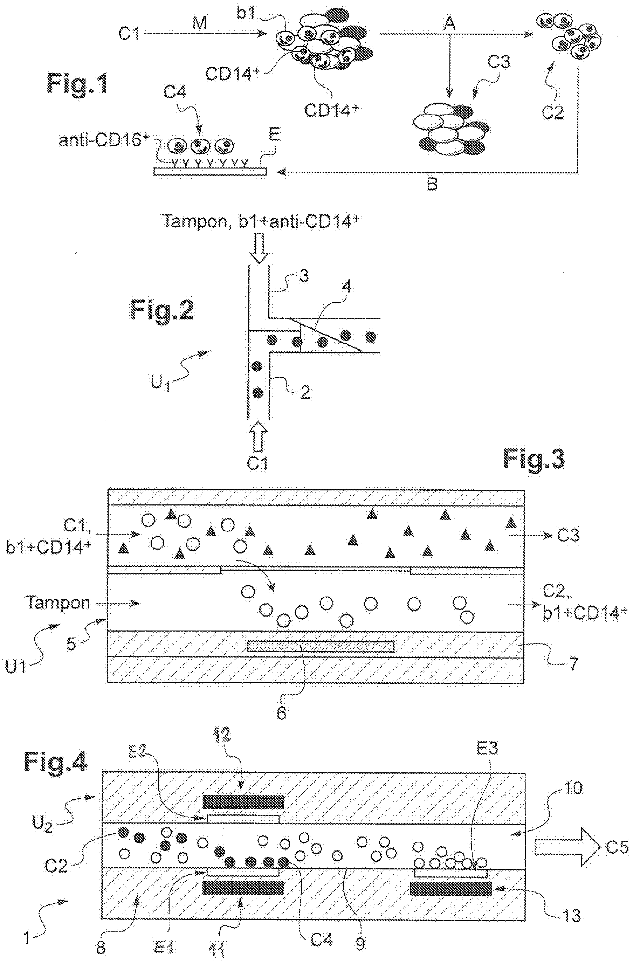

Un système microfluidique 1, 101 selon l'invention (visible partiellement aux figures 2, 3, 4, 6, 7) peut être avantageusement utilisé comme illustré à la figure 1, qui concerne l'exemple préférentiel de l'invention mettant en oeuvre un tri immunologique des cellules d'intérêt suivant plusieurs antigènes. Afin de mieux comprendre le principe de l'invention, l'exemple du tri et du comptage simultané des monocytes infectés a été pris, étant précisé que cet exemple n'est pas limitatif.

Une première unité de tri U₁ (visible aux figures 2 et 3) est destinée par un premier tri A à éliminer au mieux les constituants d'un échantillon C1 de sang total afin de n'en conserver que les monocytes C2 formant les cellules d'intérêt dans cet exemple. On effectue ce tri A suite à une étape de marquage M au moyen de micro- ou nanobilles magnétiques b1 fonctionnalisées par des anticorps capables de reconnaître les monocytes C2 (comme par exemple des anticorps *anti-CD14* pouvant reconnaitre l'antigène correspondant *CD14* exprimé par tous les monocytes C2). On réalise le mélange bille/cellules du sang sur le dispositif 1, 101 par l'intermédiaire de microcanaux en créneaux, par exemple. Les monocytes C2 ainsi marqués sont ensuite retenus lors d'un premier tri T par au moins une première micro-bobine 6 alimentée électriquement et agencée sous un microcanal de premier tri 5 (voir figure 3), alors que les autres constituants C3 du sang total C1 (tels que les granulocytes, les lymphocytes, les globules rouges, notamment) sont élués.

Les monocytes marqués C2 sont alors libérés et emmenés vers une seconde unité de tri et de comptage U₂ (visible aux figures 4 et 6-7), où ils sont triés plus spécifiquement (seconde étape de tri B) en fonction d'autres antigènes exprimés à leur surface (comme par exemple l'antigène *CD16* exprimé par les monocytes infectés C4 formant la sous-population à isoler et à quantifier) et comptés. Suivant l'application envisagée, plusieurs antigènes peuvent être ciblés en parallèle.

Dans cette seconde unité U₂, les monocytes infectés C4 sont piégés à l'intérieur d'un microcanal de second tri 10 sur une électrode fonctionnalisée E (voir figures 1, 5, 6 et 7) ou bien sur une paire d'électrodes fonctionnalisées E1 et E2 en regard dans l'exemple de la figure 4, la fonctionnalisation étant réalisée par un anticorps capable de se lier avec un antigène d'intérêt (par exemple un anticorps *anti-CD16* pour l'antigène *CD16* des monocytes infectés C2). Pour maximiser le piégeage, on installe une unique seconde micro-bobine 10a en dessous de l'électrode E ou bien, selon l'invention, une paire de secondes micro-bobines 11 et 12 alimentées électriquement respectivement autour des électrodes fonctionnalisées en regard E1 et E2, afin d'attirer les monocytes infectés C4 sur les électrodes E ou E1, E2 et ainsi d'augmenter leur probabilité d'interaction avec leur surface fonctionnalisée. Les cellules non spécifiquement accrochées sur ces électrodes E ou E1, E2 sont ensuite relâchées et éventuellement attirées sur d'autres électrodes fonctionnalisées par d'autres anticorps, en fonction de l'application souhaitée.

On compte les monocytes infectés C4 ou, de manière plus générale, la ou chaque sous-population C4 de cellules piégée sur l'électrode fonctionnalisée correspondante grâce à propriété électriquement isolante de cette sous-population C4, par spectroscopie d'impédance électrochimique.

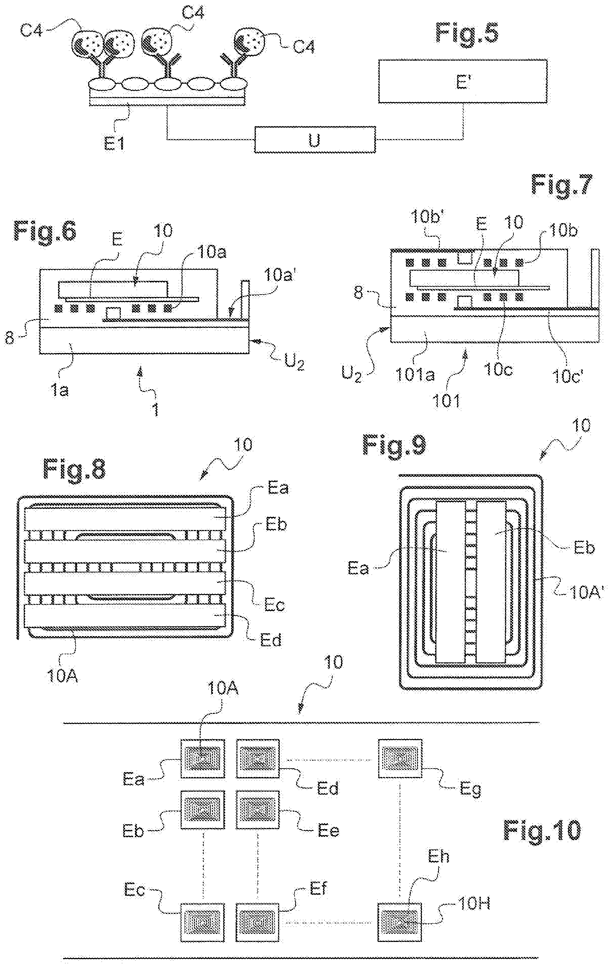

Les figures 6 et 7 montrent en coupe transversale deux systèmes microfluidiques 1, 101 selon l'invention qui comprennent un substrat 1a, 101a et qui intègrent :
- pour celui de la figure 6, la ou chaque électrode fonctionnalisée E (par exemple en or) revêtant la face interne 9 du ou de chaque microcanal 10 et associée à une unique micro-bobine 10a par exemple en cuivre qui est intégrée à la paroi 8 du microcanal 10 en dessous de cette face interne 9, et
- pour celui de la figure 7, la ou chaque électrode E (par exemple en or) qui est associée à deux micro-bobines 10b et 10c par exemple également en cuivre, ces micro-bobines 10b et 10c étant intégrées à la paroi 8 du ou de chaque microcanal 10 de part et d'autre de ce dernier (i.e. au-dessus et en dessous des côtés supérieur et inférieur de sa face interne 9 de section rectangulaire).

La paroi 8 du ou de chaque microcanal 10 est par exemple réalisée en un polymère photosensible (e.g. de type SU8). De plus, sont visibles aux figures 6 et 7 les connexions électriques 10a', 10b', 10c' (e.g. en cuivre) respectivement pour l'alimentation des micro-bobines 10a, 10b, 10c.

Plus précisément en relation avec la première unité de tri U₁, on voit à la figure 2 que l'étape M de marquage des cellules d'intérêt C2 (e.g. des monocytes) est mise en oeuvre par mélange au moyen de deux microcanaux séparés 2 et 3 qui véhiculent respectivement, dans le microcanal 2 inférieur, la population de cellules C1 à analyser (e.g. le sang total) et, dans le microcanal 3 supérieur, une solution tampon contenant les micro- ou nanobilles magnétiques b1 préalablement fonctionnalisées en surface par les anticorps par exemple *anti-CD14.* On peut utiliser au choix des mélangeurs actifs ou passifs, ainsi qu'optionnellement une encapsulation des billes b1 et des cellules dans des gouttes, par exemple. Ces microcanaux 2 et 3 se rejoignent en une zone de jonction 4 débouchant dans le microcanal de premier tri 5 visible à la figure 3 au sein de la première unité de tri U₁.

Est ainsi visible à la figure 3 la mise en oeuvre du tri A par une attraction magnétique, laquelle est réalisée au moyen de la micro-bobine 6 qui est agencée hors du microcanal 5, par exemple en étant intégrée à la paroi fermée 7 de ce microcanal 5. L'activation de la micro-bobine 6 permet le piégeage des complexes billes b1/ monocytes C2.

On rince ensuite le ou chaque microcanal de premier tri 5 de manière à éliminer tous les constituants du sang C3 autres que les monocytes ainsi piégés C2. Cette étape de purification permet de façon simple d'éliminer toutes les espèces non désirées, pour obtenir une solution dont les propriétés physico-chimiques sont connues et contrôlées. Après ce rinçage, les complexes billes b1/ monocytes C2 sont relâchés en solution et sont entraînés par l'écoulement vers la seconde unité de tri et de comptage U₂.

On voit à l'exemple de la figure 4 que cette unité U₂ comprend la paire d'électrodes E1 et E2 fonctionnalisées sur la face interne 9 de la paroi fermée 8 d'un microcanal de second tri 10, et la paire de micro-bobines 11 et 12 associée afin d'attirer, parmi l'ensemble des complexes marqués, les monocytes infectés C4 sur ces électrodes E1 et E2. Ces micro-bobines 11 et 12 sont par exemple intégrées à la paroi 8 (étant ainsi disposées hors de l'espace interne au microcanal 10) et attirent ces monocytes infectés C4 exprimant les antigènes *CD16* en contact étroit avec la surface de ces électrodes fonctionnalisées dans cet exemple par les anticorps *anti-CD16*. Lors de la désactivation des micro-bobines 11 et 12, seuls les monocytes exprimant les antigènes *CD16* et ayant établi des interactions antigène-anticorps restent fixés sur les électrodes E1 et E2, les autres monocytes C5 étant emportés par l'écoulement en aval du microcanal 10. Après ce piégeage des monocytes infectés, leur nombre est comptabilisé directement par la technique précitée de spectroscopie d'impédance.

D'une manière générale, on notera que la probabilité de piégeage de la ou de chaque sous-population à quantifier, telle que ces monocytes infectés, peut être maximisée de différentes manières. En particulier, l'agencement des deux micro-bobines 11 et 12 respectivement dessous et dessus le microcanal 10 permet de réaliser des cycles d'attraction-libération successifs et alternés entre les deux micro-bobines 11 et 12 pour l'obtention d'une efficacité de piégeage maximale. En variante ou en combinaison avec cet agencement préférentiel d'électrodes E1 et E2 en regard, on peut prévoir plusieurs électrodes fonctionnalisées et bobines associées qui sont disposées les unes à la suite des autres, afin de « rattraper » les cellules qui n'auraient pas été piégées par ces électrodes fonctionnalisées.

Enfin et de manière à connaître le ratio des monocytes *CD16*+ / *CD14*+*,* le ou chaque microcanal de second tri 10 selon l'invention peut être en outre pourvu, toujours sur la face interne 9 de sa paroi 8, d'au moins une ultime électrode non fonctionnalisée E3 couplée à au moins une ultime micro-bobine 13 en regard disposée dans cette paroi 8, hors de l'espace interne au microcanal 10. De cette manière, il est possible d'immobiliser par piégeage magnétique sur cette électrode E3 les monocytes *CD14*+ non piégés immunologiquement par les électrodes E1 et E2 prévues en amont, et de compter ces monocytes CD14+ par la même technique de spectroscopie d'impédance.

La figure 5 illustre cette technique de comptage par spectroscopie d'impédance électrochimique (SIE), qui consiste à mesurer l'impédance de l'interface entre la ou chaque électrode fonctionnalisée E1, E2 et la couche de cellules piégées à sa surface (e.g. couche de monocytes infectés C4), en imposant une faible différence de potentiel U alternative et en mesurant le courant qui en résulte à différentes fréquences, au moyen d'une contre-électrode ou électrode de référence E'. L'impédance Z est donnée par le rapport tension U / courant I et est donnée de manière connue par la formule Z = U /I = | Z | (cos φ + i sin φ).

La spectroscopie d'impédance électrochimique est particulièrement intéressante dans le cas de couches cellulaires, les cellules ayant d'excellentes propriétés électriquement isolantes. Un changement d'impédance peut provenir soit d'un changement du taux de couverture de l'électrode E, E1, E2 (c'est le cas lorsqu'une cellule adhère, grossit, meurt ou migre à la surface de l'électrode), soit d'une variation du caractère électriquement isolant de la couche cellulaire C4, Par conséquent, en mesurant l'impédance Z à l'interface cellules C4 / électrode E, E1, E2 pour une large gamme de fréquences, il est possible, par le biais de la modélisation des diagrammes obtenus (diagrammes de Nyquist Zim = f (Zr)) en circuit électrique équivalent composés de résistances et de capacités, de remonter au taux de couverture des cellules et donc au nombre de cellules C4 piégées.

Deux méthodes distinctes existent pour ces mesures :
- les mesures d'impédance réalisées en présence d'une espèce redox qui joue le rôle de sonde (Fe(CN)₆^{3-/4-} par exemple). Dans ce cas, la capacité de la sonde à aller se réduire ou s'oxyder à l'électrode E, E1, E2 est déduite des mesures d'impédance en évaluant la résistance au transfert de charge qui varie en fonction du caractère plus ou moins électriquement isolant de la couche cellulaire déposée. On parle dans ce cas d'impédance faradique : et
- les mesures réalisées dans un milieu « neutre » (e.g. dans une solution tampon ou un milieu de culture) qui permettent d'accéder aux phénomènes se produisant au niveau du film cellulaire. On parle dans ce cas d'impédance non faradique, et c'est dans ce cas la résistance du film qui évolue en présence des cellules piégées.

Dans la présente invention, c'est la mesure d'impédance en mode non faradique qui est préférentiellement mise en oeuvre pour le comptage des cellules C4 piégées sur l'électrode E1, E2, du fait que cette mesure s'avère plus sensible.

Les figures 8-11 illustrent des exemples d'agencement d'électrodes fonctionnalisées Ea, Eb, Ec, Ed, Ee, Ef, Eg, Eh, etc. dans un système microfluidique 1, 101 selon l'invention. Ces électrodes Ea, ..., Eh peuvent être prévues dans des microcanaux 10 séparés mais également dans un même microcanal 10.

A la figure 8, les électrodes Ea, Eb, Ec, Ed, fonctionnalisées de façon identique ou non, sont agencées en parallèle en étant associées à une micro-bobine de piégeage collective 10A (on pourrait utiliser selon l'invention des micro-bobines de piégeage individuelles).

A la figure 9, les électrodes Ea, Eb, fonctionnalisées de façon identique ou non, sont agencées en série en étant associées à une micro-bobine de piégeage collective 10A' (on pourrait utiliser selon l'invention des micro-bobines de piégeage individuelles).

A la figure 10, les électrodes Ea,..., Eh, fonctionnalisées à l'identique ou non, sont agencées en matrice, adressées individuellement et associées à des micro-bobines de piégeage individuelles 10A, ...., 10H.

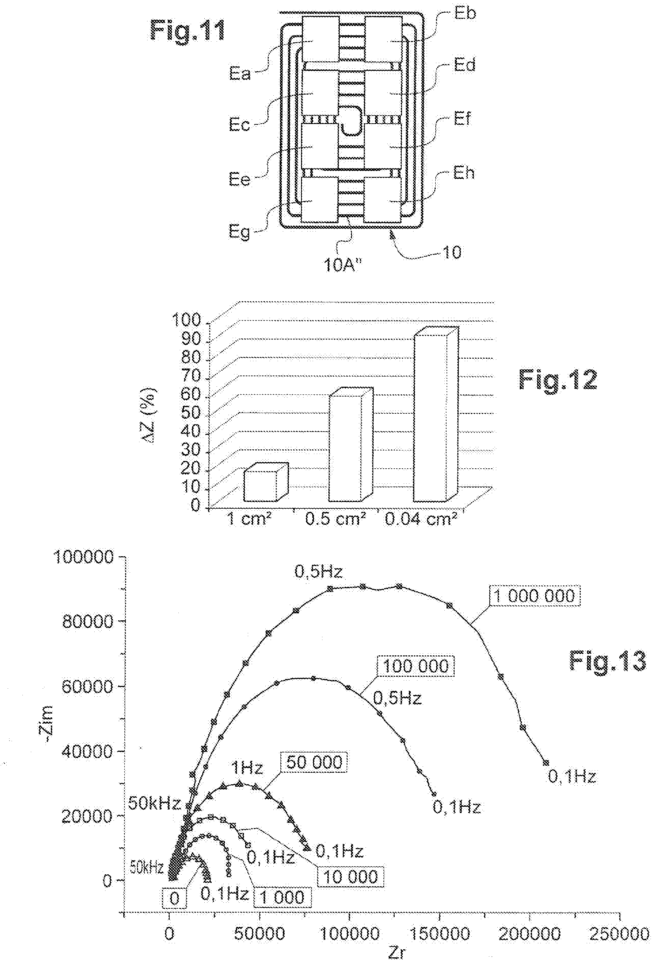

A la figure 11, les électrodes Ea, .,., Eh, fonctionnalisées à l'identique ou non, sont agencées en matrice, adressées individuellement et associées à une micro-bobine de piégeage collective 10A".

Selon la taille de chaque électrode Ea, ..., Eh, on peut envisager un fonctionnement « analogique » (signal obtenu proportionnel au nombre de cellules piégées) ou « digital », En effet, l'électrode ayant une taille de l'ordre de grandeur de celle de la cellule, elle ne détecte alors qu'une cellule à la fois et le signal sera donc binaire : 1 ou 0.

La Demanderesse a réalisé des essais portant sur la sensibilité du comptage par spectroscopie d'impédance électrochimique au niveau de chaque électrode E, E1, E2, en fonction de l'aire de celle-ci (figure 12), ainsi que sur la variation d'impédance mesurée en fonction du nombre de cellules piégées sur chacune de ces électrodes E, E1, E2,

Le graphique de la figure 12 montre que la sensibilité de la mesure pour le comptage de chaque sous-population de cellules C4 selon l'invention (variation d'impédance mesurée en % pour ce comptage) est d'autant plus élevée que l'aire de chaque électrode fonctionnalisée de piégeage E, E1, E2 est plus réduite (aire de 0,04 cm² pour la valeur maximale de ΔZ proche de 90 %).

Le graphique de la figure 13 (avec les fréquences utilisées variant entre 0,1 Hz et 50 kHz pour les impédances les plus faibles en bas à gauche) montre que l'impédance mesurée est d'autant plus élevée que la concentration en cellules sur l'électrode E, E1, E2 est plus forte, comme le montrent les valeurs de concentrations en cellules (encadrées) qui figurant à côté de chaque courbe et qui atteignent 1 000 000 pour la courbe du haut (en comparaison de la courbe du bas relative à une concentration nulle en cellules, et des courbes intermédiaires où le nombre de cellules est successivement de 1000, 10 000, 50 000 et 100 000).

La Demanderesse a en outre fait varier, pour deux diamètres des microbilles b1 utilisées pour le marquage (2,3 µm et 4,5 µm), la pression d'écoulement (de 5 à 10 bar) et le nombre de micro-bobines actives dans le microcanal de second tri 10 (de une à trois micro-bobines, avec une intensité du courant d'alimentation des micro-bobines de 100 mA), pour comparer selon ces paramètres l'efficacité de la déviation et du piégeage ou de la séparation des seules microbilles magnétiques en fonction de la pression du flux d'écoulement dans le système microfluidique 1,101.

Les résultats obtenus sont recensés dans les tableaux ci-après.

**Tableau 1 : billes de 2,3 µm de diamètre**

| **Pression** | **Séparation** |
|---|---|
| 5 mbar | 87 % |
| 6 mbar | 86,5 % |
| 7 mbar | 73 % |

A 10 mbar, on a une vitesse d'écoulement très élevée et un piégeage nul.

**Tableau 2 ; billes de 4;5 µm de diamètre, pression de 7 mbar**

| **Nombre de micro-bobines actives** | **Piégeage** | **Séparation** |
|---|---|---|
| 1 | 80 % | 100 % |
| 2 | 100 % | 100 % |
| 3 | 100 % | 100 % |

**Tableau 3 : billes de 4,5 µm de diamètre, pression de 8 mbar**

| **Nombre de micro-bobines actives** | **Piégeage** | **Séparation** |
|---|---|---|
| 1 | 0 % | 74 % |
| 2 | 32 % | 71 % |
| 3 | 42 % | 88 % |

Ces résultats montrent notamment l'avantage d'utiliser au moins deux micro-bobines actives et une pression d'écoulement comprise entre 5 mbar et 8 mbar environ, pour mettre en oeuvre la méthode d'isolement et de comptage selon l'invention.

## Revendications

1. Système microfluidique (1, 101) apte à recevoir des populations de cellules (C1) et apte à isoler et à quantifier simultanément pour chacune desdites populations au moins une sous-population de cellules (C4), ledit système comportant un substrat (1a, 101a) dans lequel est gravé un réseau de microcanaux (2, 3, 4, 5, 10) comprenant une première unité de tri (U₁) apte à isoler par attraction magnétique des cellules d'intérêt (C2) de ladite population de cellules dans au moins un microcanal de premier tri (5),
ledit réseau comprenant une seconde unité de tri et de comptage simultanés (U₂) comportant au moins un microcanal de second tri (10) qui communique directement ou indirectement avec ledit au moins un microcanal de premier tri et qui est défini par une paroi fermée (8) présentant une face interne (9) destinée à être en contact avec lesdites cellules d'intérêt, ladite face interne étant pourvue d'au moins une électrode fonctionnalisée (E, E1, E2, Ea, ..., Eh) apte à piéger spécifiquement une dite sous-population de cellules (C4) parmi lesdites cellules d'intérêt. ladite seconde unité de tri et de comptage comportant des moyens de comptage (E, E1) E2, Ea,..., Eh, E') par spectroscopie d'impédance électrochimique de ladite ou chaque sous-population piégée sur ladite au moins une électrode fonctionnalisée,
**caractérisé en ce que** ledit au moins un microcanal de second tri (10) est pourvu d'au moins une paire de dites électrodes fonctionnalisées (E1 et E2) agencées en regard l'une de l'autre en deux côtés opposés de ladite face interne (9), et d'au moins une paire associée de secondes micro-bobines de piégeage (11 et 12) alimentées électriquement qui sont aptes à amener lesdites cellules d'intérêt (C2) en contact étroit avec lesdites électrodes fonctionnalisées, qui sont agencées dans ladite paroi (8) de manière adjacente et en regard desdites électrodes respectives et qui sont aptes à commander des cycles d'attraction et de libération successifs et alternés entre les micro-bobines de ladite au moins une paire de secondes micro-bobines.

2. Système microfluidique (1, 101) selon la revendication 1, **caractérisé en ce que** ladite première unité de tri (U₁) comprend :
- un microcanal d'amenée (2) d'une dite population de cellules (C1) qui débouche dans ledit au moins un microcanal de premier tri (5),
- un microcanal d'introduction (3) de moyens magnétiques de marquage (b1, anti CD14) desdites cellules d'intérêt (C2) qui débouche dans ledit au moins un microcanal de premier tri et dans lequel sont introduits lesdits moyens magnétiques de marquage capables de se lier spécifiquement avec lesdites cellules d'intérêt pour former des complexes magnétiques marqués (b1, C2), et
- des moyens de piégeage magnétique (6) qui sont agencés sous ledit au moins un microcanal de premier tri et qui sont capables d'attirer et de retenir lesdites complexes magnétiques marqués.

3. Système microfluidique (1, 101) selon la revendication 2, **caractérisé en ce que** lesdits moyens magnétiques de marquage (b1, anti CD14) comprennent des microbilles ou des nanobilles magnétiques (b1) fonctionnalisées par une première substance (anti-CD14) apte à reconnaître spécifiquement un premier marqueur (CD14) desdites cellules d'intérêt (C2), et **en ce que** lesdits moyens de piégeage magnétique (6) comprennent au moins une première micro-bobine (6) alimentée électriquement

4. Système microfluidique (1, 101) selon une des revendications précédentes, **caractérisé en ce que** lesdites électrodes fonctionnalisées (E1, E2) sont fonctionnalisées par une seconde substance (anti-CD16) capable de se lier spécifiquement avec un second marqueur (CD16) de ladite sous-population (C4) à isoler parmi lesdites cellules d'intérêt (C2).

5. Système microfluidique (1, 101) selon une des revendications précédentes, **caractérisé en ce que** ladite face interne (9) de paroi (8) dudit au moins un microcanal de second tri (10) est pourvue de manière espacée sur sa longueur d'une succession de plusieurs électrodes (E1 et E2, E3) incluant ladite au moins une paire d'électrodes fonctionnalisées (E1 et E2) puis une électrode non fonctionnalisée ou paire d'électrodes non fonctionnalisées (E3) agencées en regard l'une de l'autre en deux côtés opposés de ladite face interne, pour réaliser des isolements et quantifications en série de plusieurs dites sous-populations de cellules (C4).

6. Système microfluidique (1, 101) selon la revendication 5, **caractérisé en ce que** ladite seconde unité de tri et de comptage (U₂) comprend une pluralité de dites paires d'électrodes fonctionnalisées (Ea et Eb) qui sont agencées en série sur un même dit microcanal de second tri (10) ou bien sur plusieurs dits microcanaux de second tri, qui sont fonctionnalisées de manière identique ou non et qui sont associées à plusieurs dites paires de secondes micro-bobines de piégeage individuelles.

7. Système microfluidique (1, 101) selon une des revendications précédentes, **caractérisé en ce que** ladite première unité de tri (U₁) comprend une pluralité de dits microcanaux premier tri (5) et **en ce que** ladite seconde unité de tri et de comptage (U₂) comprend une pluralité de dits microcanaux de second tri (10) qui sont chacun pourvus de ladite au moins une paire d'électrodes fonctionnalisées (E1, E2, Ea, ..., Eh), de telle sorte que ledit système réalise en parallèle une pluralité d'isolements et de quantifications différents de dites sous-populations de cellules (C4) à partir de différentes cellules d'intérêt (C2).

8. Système microfluidique (1, 101) selon la revendication 7, **caractérisé en ce que** ladite seconde unité de tri et de comptage (U₂) comprend une pluralité de dites paires d'électrodes fonctionnalisées (Ea, Eb, Ec, Ed) qui sont agencées en parallèle sur un même dit microcanal de second tri (10) ou bien sur plusieurs dits microcanaux de second tri, qui sont fonctionnalisées de manière identique ou non et qui sont associées à autant de dites paires de secondes micro-bobines de piégeage individuelles.

9. Système microfluidique (1, 101) selon une des revendications précédentes, **caractérisé en ce que** ladite seconde unité de tri et de comptage (U₂) comprend une matrice de dites paires d'électrodes fonctionnalisées (Ea, ..., Eh) qui sont adressées individuellement, et qui sont associées à autant de dites secondes micro-bobines de piégeage individuelles (10A, ..., 10H).

10. Procédé d'isolement et de quantification simultanés d'au moins une sous-population de cellules (C4) à partir d'une population de cellules (C1), **caractérisé en ce qu'**il comprend un écoulement d'un échantillon comprenant ladite population de cellules dans un réseau de microcanaux (2, 3, 4, 5,10) d'un système microfluidique (1, 101) selon une des revendications précédentes, avec les étapes successives suivantes :
a) un piégeage par attraction magnétique desdites cellules d'intérêt (C2) dans ledit au moins un microcanal de premier tri (5),
b) une élimination des constituants indésirables dudit échantillon pour ne conserver que lesdites cellules d'intérêt ainsi piégées, puis
c) un piégeage de ladite au moins une sous-population de cellules parmi lesdites cellules d'intérêt dans ledit au moins un microcanal de second tri (10) par ladite au moins une paire d'électrodes fonctionnalisées (E1, E2), et un comptage concomitant par spectroscopie d'impédance électrochimique de ladite au moins une sous-population piégée.

11. Procédé d'isolement et de quantification selon la revendication 10, **caractérisé en ce qu'**à l'étape a) :
- on marque lesdites cellules d'intérêt (C2) par des microbilles ou nanobilles magnétiques (b1) fonctionnalisées par une première substance (anti-CD14) se liant spécifiquement avec un premier marqueur (CD14) desdites cellules d'intérêt, pour l'obtention de complexes magnétiques marqués (b1, C2), puis
- on piège magnétiquement lesdits complexes par au moins une première micro-bobine (6).

12. Procédé d'isolement et de quantification selon la revendication 11, **caractérisé en ce qu'**à l'étape c), on attire lesdits complexes magnétiques marqués (b1, C2) par ladite au moins une paire de secondes micro-bobines de piégeage (11, 12, 10a, 10b, 10c) qui est agencée en regard de ladite au moins paire d'électrodes fonctionnalisées (E1, E2) par une seconde substance (anti-CD16) se liant spécifiquement avec un second marqueur (CD16) de ladite au moins une sous-population (C4) à isoler, et qui amène lesdits complexes en contact étroit avec ladite au moins une paire d'électrodes fonctionnalisées pour le piégeage et le comptage de cette sous-population.

13. Procédé d'isolement et de quantification selon la revendication 12, **caractérisé en ce que** ledit échantillon contenant ladite population de cellules (C1) est un échantillon sang total lesdites cellules d'intérêt (C2) étant des monocytes que l'on isole magnétiquement à l'étape a) avec ledit premier marqueur exprimé par les cellules d'intérêt qui est un antigène par exemple CD14 et ladite première substance de marquage qui est un anticorps par exemple anti-CD14, ladite sous-population de cellules (C4) isolée et quantifiée par une tri immunologique à l'étape c) comprenant des monocytes infectés (C4) exprimant ledit second marqueur qui est un antigène par exemple CD16, ladite seconde substance étant un anticorps par exemple anti-CD16.

14. Procédé d'isolement et de quantification selon une des revendications 10 à 13, **caractérisé en ce que** le comptage par spectroscopie d'impédance électrochimique de l'étape c) est réalisé en mode non faradique ou faradique.

## Patentansprüche

1. Mikrofluidisches System (1, 101), das imstande ist, Zellpopulationen (C1) zu empfangen und imstande, für jede der Populationen mindestens eine Unter-Zellpopulation (C4) gleichzeitig zu isolieren und zu quantifizieren, wobei das System ein Substrat (1a, 101a) aufweist, in welches ein Mikrokanalnetz (2, 3, 4, 5, 10) graviert ist, umfassend eine erste Sortiereinheit (U₁), die imstande ist, durch magnetische Anziehung interessierende Zellen (C2) der Zellpopulation in mindestens einem Mikrokanal erster Sortierung (5) zu isolieren,
wobei das Netz eine zweite Einheit (U₂) umfasst, gleichzeitig sortiert und zählt, die mindestens einen Mikrokanal zweiter Sortierung (10) aufweist, der direkt oder indirekt mit dem mindestens einen Mikrokanal erster Sortierung kommuniziert und der durch eine geschlossene Wand (8) definiert ist, die eine Innenseite (9) aufweist, die bestimmt ist, mit den interessierenden Zellen im Kontakt zu sein, wobei die Innenseite mit mindestens einer funktionalisierten Elektrode (E, E1, E2, Ea, ..., Eh) ausgestattet ist, die imstande ist, speziell eine Unter-Zellpopulation (C4) aus den interessierenden Zellen einzufangen,
wobei die zweite Sortier- und Zähleinheit Zählmittel (E, E1, E2, Ea, ..., Eh, E') durch elektrochemische Impedanzspektroskopie der oder jeder auf der mindestens einen funktionalisierten Elektrode eingefangenen Unterpopulation aufweist,
**dadurch gekennzeichnet, dass** der mindestens eine Mikrokanal zweiter Sortierung (10) mit mindestens einem Paar funktionalisierter Elektroden (E1 und E2) ausgestattet ist, die an zwei gegenüberliegenden Seiten der Innenseite (9) einander zugewandt ausgebildet sind, und mit mindestens einen zugeordneten Paar zweiter elektrisch versorgter Mikrofangspulen (11 und 12), die imstande sind, die interessierenden Zellen (C2) in engen Kontakt mit den funktionalisierten Elektroden zu führen, die in der Wand (8) neben und gegenüber den jeweiligen Elektroden ausgebildet sind und die imstande sind, aufeinanderfolgende und abwechselnde Anziehungs- und Freisetzungszyklen zwischen den Mikrospulen des mindestens einen zweiten Mikrospulenpaars zu steuern.

2. Mikrofluidisches System (1, 101) nach Anspruch 1, **dadurch gekennzeichnet, dass** die erste Sortiereinheit (U₁) umfasst:
- einen Mikrozufuhrkanal (2) einer Zellpopulation (C1), der in den mindestens einen Mikrokanal erster Sortierung (5) ausmündet,
- eine Mikroeinleitkanal (3) magnetischer Markermittel (b1, Anti-CD14) der interessierenden Zellen (C2), der in den mindestens einen Mikrokanal erster Sortierung ausmündet und in den die magnetischen Markermittel eingeführt werden, die imstande sind, sich speziell mit den interessierenden Zellen zu vereinen, um markierte magnetische Komplexe (b1, C2) zu bilden, und
- magnetische Einfangmittel (6), die unter dem mindestens einen Mikrokanal erster Sortierung ausgebildet sind und die imstande sind, die markierten magnetischen Komplexe anzuziehen und zu halten.

3. Mikrofluidisches System (1, 101) nach Anspruch 2, **dadurch gekennzeichnet, dass** die magnetischen Markermittel (b1, Anti-CD14) magnetische Mikrokugeln oder Nanokugeln (b1) umfassen, die durch eine erste Substanz (Anti-CD14) funktionalisiert sind, die imstande ist, spezifisch einen ersten Marker (CD14) der interessierenden Zellen (C2) zu erkennen, und dass die magnetischen Einfangmittel (6) mindestens eine erste, elektrisch versorgte Mikrospule (6) umfassen.

4. Mikrofluidisches System (1, 101) nach einem der vorangehenden Ansprüche, **dadurch gekennzeichnet, dass** die funktionalisierten Elektroden (E1, E2) mit einer zweiten Substanz (Anti-CD16) funktionalisiert sind, die imstande ist, sich speziell mit einem zweiten Marker (CD16) der zu isolierenden Unterpopulation (C4) der interessierenden Zellen (C2) zu vereinen.

5. Mikrofluidisches System (1, 101) nach einem der vorangehenden Ansprüche, **dadurch gekennzeichnet, dass** die Innenseite (9) der Wand (8) des mindestens einen Mikrokanals zweiter Sortierung (10) über seine Länge beabstandet mit einer Abfolge mehrerer Elektroden (E1 und E2, E3), das mindestens ein Paar funktionalisierter Elektroden (E1 und E2), dann eine nicht funktionalisierte Elektrode oder ein Paar nicht funktionalisierter Elektroden (E3) einschließend, ausgestattet ist, die an zwei gegenüberliegenden Seiten der Innenseite einander zugewandt ausgebildet sind, um serienmäßig Isolierungen und Quantifizierungen mehrerer Unter-Zellpopulationen (C4) durchzuführen.

6. Mikrofluidisches System (1, 101) nach Anspruch 5, **dadurch gekennzeichnet, dass** die zweite Sortier- und Zähleinheit (U₂) eine Vielzahl von Paaren funktionalisierter Elektroden (Ea und Eb) umfasst, die in Reihe auf einem selben Mikrokanal zweiter Sortierung (10) oder auch auf mehreren Mikrokanälen zweiter Sortierung angeordnet sind, die identisch oder nicht identisch funktionalisiert sind und die mehreren Paaren individueller zweiter Mikrofangspulen zugeordnet sind.

7. Mikrofluidisches System (1, 101) nach einem der vorangehenden Ansprüche, **dadurch gekennzeichnet, dass** die erste Sortiereinheit (U₁) eine Vielzahl von Mikrokanälen erster Sortierung (5) umfasst und dass die zweite Sortier- und Zähleinheit (U₂) eine Vielzahl von Mikrokanälen zweiter Sortierung (10) umfasst, die jeweils mit dem mindestens einen Paar funktionalisierter Elektroden (E1, E2, Ea, ..., Eh) ausgestattet sind, so dass das System parallel eine Vielzahl verschiedener Isolierungen und Quantifizierungen von Unter-Zellpopulationen (C4) auf der Basis verschiedener interessierender Zellen (C2) durchführt.

8. Mikrofluidisches System (1, 101) nach Anspruch 7, **dadurch gekennzeichnet, dass** die zweite Sortier- und Zähleinheit (U₂) eine Vielzahl von Paaren funktionalisierter Elektroden (Ea, Eb, Ec, Ed) umfasst, die parallel auf einem selben Mikrokanal zweiter Sortierung (10) oder auch auf mehreren Mikrokanälen zweiter Sortierung angeordnet sind, die identisch oder nicht identisch funktionalisiert sind und die ebenso vielen Paaren individueller zweiter Mikrofangspulen zugeordnet sind.

9. Mikrofluidisches System (1, 101) nach einem der vorangehenden Ansprüche, **dadurch gekennzeichnet, dass** die zweite Sortier- und Zähleinheit (U₂) eine Matrix von Paaren funktionalisierter Elektroden (Ea, ..., Eh) umfasst, die individuell adressiert sind, und die ebenso vielen individuellen zweiten Mikrofangspulen (10A, ..., 10H) zugeordnet sind.

10. Verfahren für die gleichzeitige Isolierung und Quantifizierung mindestens einer Unter-Zellpopulation (C4) auf der Basis einer Zellpopulation (C1), **dadurch gekennzeichnet, dass** es einen Strom einer Probe, umfassend die Zellpopulation, in einem Mikrokanalnetz (2, 3, 4, 5, 10) eines mikrofluidischen Systems (1, 101) nach einem der vorangehenden Ansprüche umfasst, mit den folgenden aufeinanderfolgenden Schritten:
a) Einfangen durch magnetische Anziehung der interessierenden Zellen (C2) in dem mindestens einen Mikrokanal erster Sortierung (5),
b) Entfernen der unerwünschten Bestandteile aus der Probe, um nur die derart eingefangenen interessierenden Zellen zu behalten, dann
c) Einfangen der mindestens einen Unter-Zellpopulation der interessierenden Zellen in dem mindestens einen Mikrokanal zweiter Sortierung (10) durch das mindestens eine Paar funktionalisierter Elektroden {E1, E2) und begleitendes Zählen durch elektrochemische Impedanzspektroskopie der mindestens einen eingefangenen Unterpopulation.

11. Isolations- und Quantifizierungsverfahren nach Anspruch 10, **dadurch gekennzeichnet, dass** in Schritt a):
- die interessierenden Zellen (C2) mit magnetischen Mikrokugeln oder Nanokugeln (b1) markiert werden, die mit einer ersten Substanz (Anti-CD14) funktionalisiert sind, die sich speziell mit einem ersten Marker (CD14) der interessierenden Zellen verbindet, um markierte magnetische Komplexe (b1, C2) zu erhalten, dann
- die Komplexe mit mindestens einer ersten Mikrospule (6) magnetisch eingefangen werden.

12. Isolations- und Quantifizierungsverfahren nach Anspruch 11, **dadurch gekennzeichnet, dass** in Schritt c) die markierten magnetischen Komplexe (b1, C2) von dem mindestens einen Paar zweiter Mikrofangspulen (11, 12, 10a, 10b, 10c) angezogen werden, das gegenüber dem mindestens einen Paar Elektroden (E1, E2) angeordnet ist, die von einer zweiten Substanz (Anti-CD16) funktionalisiert sind, die sich speziell mit einem zweiten Marker (CD16) der mindestens einen zu isolierenden Unterpopulation (C4) verbindet, und das die Komplexe für das Einfangen und das Zählen dieser Unterpopulation in engen Kontakt mit dem mindestens einen Paar funktionalisierter Elektroden führt.

13. Isolations- und Quantifizierungsverfahren nach Anspruch 12, **dadurch gekennzeichnet, dass** die Probe, welche die Zellpopulation (C1) enthält, eine Vollblutprobe ist, wobei die interessierenden Zellen (C2) Monocyten sind, die in Schritt a) mit dem ersten Marker, der von den interessierenden Zellen exprimiert wird, der ein Antigen, beispielsweise CD14, ist, und der ersten Markersubstanz, die ein Antikörper, beispielsweise Anti-CD14, ist, magnetisch isoliert werden, wobei die mittels einer immunologischen Sortierung in Schritt c) isolierte und quantifizierte Unter-Zellpopulation (C4) infizierte Monocyten (C4) umfasst, die den zweiten Marker exprimieren, der ein Antigen, beispielsweise CD16, ist, wobei die zweite Substanz ein Antikörper, beispielsweise Anti-CD16, ist.

14. Isolations- und Quantifizierungsverfahren nach einem der Ansprüche 10 bis 13, **dadurch gekennzeichnet, dass** das Zählen durch elektrochemische Impedanzspektroskopie von Schritt c) im Nicht-Faradayschen oder im Faradayschen Modus durchgeführt wird.

## Claims

1. A microfluidic system (1, 101) capable of receiving populations of cells (C1) and capable of isolating and quantifying simultaneously for each of said populations at least one sub-population of cells (C4), said system including a substrate (1a, 101a) in which is etched a network of microchannels (2, 3, 4, 5, 10) comprising a first sorting unit (U₁) capable of isolating by magnetic attraction of the cells of interest (C2) of said population of cells in at least one first sorting microchannel (5),
said network comprising a second simultaneous sorting and counting unit (U₂) including at least one second sorting microchannel (10) which directly or indirectly communicates with said at least one first sorting microchannel and which is defined by a closed wall (8) having an internal face (9) intended to be in contact with said cells of interest, said internal face being provided with at least one functionalized electrode (E, E1, E2, Ea, ..., Eh) capable of specifically trapping a so-called sub-population of cells (C4) from among said cells of interest,
said second sorting and counting unit including counting means (E, E1, E2, Ea, ..., Eh, E') by electrochemical impedance spectroscopy of said or each sub-population trapped on said at least one functionalized electrode,
**characterized in that** said at least one second sorting microchannel (10) is provided with at least one pair of said functionalized electrodes (E1 and E2) laid out facing each other on two opposite sides of said internal face (9), and with at least one associated pair of second electrically powered trapping micro-coils (11 and 12) which are capable of bringing said cells of interest (C2) in close contact with said functionalized electrodes, which are laid out in said wall (8) in an adjacent way and facing said respective electrodes and which are capable of commanding successive and alternating attraction and release cycles between the micro-coils of said at least one pair of second micro-coils.

2. The microfluidic system (1, 101) according to claim 1, **characterized in that** said first sorting unit (U₁) comprises:
- a microchannel (2) for bringing a so-called population of cells (C1) which opens into said at least one first sorting microchannel (5),
- a microchannel (3) for introducing magnetic marking means (b1, anti-CD14) of said cells of interest (C2) which opens into said at least one first sorting microchannel and in which are introduced said magnetic marking means capable of specifically binding with said cells of interest in order to form marked magnetic complexes (b1, C2), and
- magnetic trapping means (6) which are laid out under said at least one first sorting microchannel and which are capable of attracting and retaining said marked magnetic complexes.

3. The microfluidic system (1, 101) according to claim 2, **characterized in that** said magnetic marking means (b1, anti-CD14) comprise magnetic micro-beads or nano-beads (b1) functionalized with a first substance (anti-CD14) capable of specifically recognizing a first marker (CD14) of said cells of interest (C2), and **in that** said magnetic trapping means (6) comprise at least one first electrically powered micro-coil (6).

4. The microfluidic system (1, 101) according to one of the preceding claims, **characterized in that** said functionalized electrodes (E1, E2) are functionalized with a second substance (anti-CD16) capable of specifically binding with a second marker (CD16) of said sub-population (C4) to be isolated from among said cells of interest (C2).

5. The microfluidic system (1, 101) according to one of the preceding claims, **characterized in that** said internal face (9) of the wall (8) of said at least one second sorting microchannel (10) is provided spaced out on its length, with a succession of several electrodes (E1 and E2, E3) including said at least one pair of functionalized electrodes (E1 and E2) and then a non-functionalized electrode or pair of non-functionalized electrodes (E3) laid out facing each other on two opposite sides of said internal face, in order to achieve isolations and quantifications in series of several so-called sub-populations of cells (C4).

6. The microfluidic system (1, 101) according to claim 5, **characterized in that** said second sorting and counting unit (U₂) comprises a plurality of said pairs of functionalized electrodes (Ea and Eb) which are laid out in series on a same said second sorting microchannel (10) or else on several so-called second sorting microchannels, which are either functionalized identically or not and which are associated with several said pairs of second individual trapping micro-coils.

7. The microfluidic system (1, 101) according to one of the preceding claims, **characterized in that** said first sorting unit (U₁) comprises a plurality of said first sorting microchannels (5) and **in that** said second sorting and counting unit (U₂) comprises a plurality of said second sorting microchannels (10) which are each provided with said at least one pair of functionalized electrodes (E1, E2, Ea, ..., Eh), so that said system produces in parallel a plurality of different isolations and quantifications from said sub-populations of cells (C4) from different cells of interest (C2).

8. The microfluidic system (1, 101) according to claim 7, **characterized in that** said second sorting and counting unit (U₂) comprises a plurality of said pairs of functionalized electrodes (Ea, Eb, Ec, Ed) which are laid out in parallel on a same said second sorting microchannel (10) or else on several so-called second sorting microchannels, which are either functionalized identically or not and which are associated with as many of said pairs of second individual trapping micro-coils.

9. The microfluidic system (1, 101) according to one of the preceding claims, **characterized in that** said second sorting and counting unit (U₂) comprises a matrix of said pairs of functionalized electrodes (Ea, ..., Eh) which are individually addressed, and which are associated with as many said second individual trapping micro-coils (10A, ..., 10H).

10. A method for simultaneous isolation and quantification of at least one sub-population of cells (C4) from a cell population (C1), **characterized in that** it comprises a flow of a sample comprising said cell population in a network of microchannels (2, 3, 4, 5, 10) of a microfluidic system (1, 101) according to one of the preceding claims, with the following successive steps:
a) trapping by magnetic attraction said cells of interest (C2) in said at least one first sorting microchannel (5),
b) removing the undesirable constituents of said sample in order to only retain the thereby trapped cells of interest, and then
c) trapping said at least one sub-population of cells from among said cells of interest in said at least one second sorting microchannel (10) by said at least one pair of functionalized electrodes (E1, E2), and concomitant counting by electrochemical impedance spectroscopy of said at least one trapped sub-population.

11. The isolation and quantification method according to claim 10, **characterized in that** in step a):
- said cells of interest (C2) are marked with magnetic micro-beads or nano-beads (b1) functionalized with a first substance (anti-CD14) specifically binding with a first marker (CD14) of said cells of interest, for obtaining marked magnetic complexes (b1, C2), and then
- said complexes are magnetically trapped by at least one first micro-coil (6).

12. The isolation and quantification method according to claim 11, **characterized in that** in step c), said marked magnetic complexes (b1, C2) are attracted by said at least one pair of second trapping micro-coils (11, 12, 10a, 10b, 10c) which is laid out facing said at least one pair of electrodes (E1, E2) functionalized with a second substance (anti-CD16) specifically binding with a second marker (CD16) of said at least one sub-population (C4) to be isolated, and which brings said complexes into close contact with said at least one pair of functionalized electrodes for trapping and counting this sub-population.

13. The isolation and quantification method according to claim 12, **characterized in that** said sample containing said population of cells (C1) is a full blood sample, said cells of interest (C2) being monocytes which are magnetically isolated in step a) with said first marker expressed by the cells of interest which is an antigen for example CD14 and said first marking substance which is an antibody for example an anti-CD14 antibody, said sub-population of cells (C4) isolated and quantified by immunological sorting in step c) comprising infected monocytes (C4) expressing said second marker which is an antigen for example CD16, said second substance being an antibody for example an anti-CD16 antibody.

14. The isolation and quantification method according to one of claims 10 to 13, **characterized in that** the counting by electrochemical impedance spectroscopy of step c) is carried out in a non-Faradic or Faradic mode.
